(19) Europäisches Patentamt

European Patent Office

Office européen des brevets

(11) Veröffentlichungsnummer: **0 005 529**
**B1**

(12) **EUROPÄISCHE PATENTSCHRIFT**

(21) Anmeldenummer: **79101467.3**

(22) Anmeldetag: **14.05.79**

(51) Int. Cl.³: **A 61 K 31/355,**
**A 61 K 9/14, A 23 L 1/30,**
**A 23 K 1/16**

(54) Verfahren zur Herstellung von Vitamin-E-Trockenpulver

(30) Priorität: **22.05.78 DE 2822324**

(43) Veröffentlichungstag der Anmeldung:
**28.11.79 Patentblatt 79/24**

(45) Bekanntmachung des Hinweises auf die Patenterteilung:
**07.01.81 Patentblatt 81/01**

(84) Benannte Vertragsstaaten:
**BE CH DE FR GB IT NL**

(56) Entgegenhaltungen:
**BE - A - 640 508**
**DE - B - 1 098 798**
**GB - A - 919 193**
**US - A - 3 124 510**

**CHEMICAL ABSTRACTS, Band 83, Nr. 22,**
**1. Dezember 1975, Columbus, Ohio, USA,**
**Seite 272, Zusammenfassung**
**Nr. 183395 p**

(73) Patentinhaber: **BASF Aktiengesellschaft**
**Carl-Bosch-Strasse 38**
**D - 6700 Ludwigshafen (DE)**
**Meggle Milchindustrie GmbH & Co. KG**
**Rosenheimer Strasse 10-22**
**D - 8094 Reitmehring (DE)**

(74) Vertreter: **Kuipers, Arie**
**Ahornstrasse 32**
**D - 8094 Reitmehring (DE)**
**Becker, Herbert**
**Hochriesstrasse 4**
**D - 8091 Edling (DE)**
**Tiefenbacher, Hubert, Dr.**
**Hauaeckerstrasse 49**
**D - 7022 Leinfelden-Echterdingen 3 (DE)**
**Schumacher, Horst, Dr.**
**Battenberger Weg 4**
**D - 6719 Bobenheim (DE)**

## Verfahren zur Herstellung von Vitamin-E-Trockenpulver

Die Erfindung betrifft die Herstellung eines Casein und Lactose enthaltenden Vitamin-E-Trockenpulvers.

Aus der deutschen Auslegeschrift N 4789/30 h ist bekannt, daß man Vitamin-Trockenpulver insbesondere Vitamin-A-Trockenpulver herstellen kann, wenn man Vitaminöl in einer Eiweißstoffe und Kohlenhydrate enthaltenden Lösung dispergiert und die Dispersion versprüht. Man erhält so Vitamin-Trockenpulver mit einem relativ niedrigen Gehalt an Vitaminen.

Ferner war aus der belgischen Patentschrift 589 766 bekannt, Vitamine, unter anderem Gemische, die Vitamin E enthalten, in Trockenpulver unter Verwendung eines Kolloids und eines Kohlehydrats zu überführen, wobei jedoch von einer aufwendigen Vorrichtung beim Versprühen Gebrauch gemacht werden muß, um ein Zusammenkleben der Teilchen nach dem Versprühen zu verhindern.

In US-Patent 3,608,083 wird außerdem ein Sprühverfahren beschrieben, das zu Produkten mit bis zu 60% Vitamin-E-aktiver Substanz führt. Man benötigt dazu aber eine spezielle Gelatine ohne Gelierfähigkeit und einem Molekulargewicht von etwa 9 000 bis 11 000.

Es bestand demgegenüber die Aufgabe, ein Verfahren zur Herstellung von Vitamin-E-Trockenpulver vorzuschlagen, das hohe Gehalte an Vitamin E aufweist, und ohne besondere apparative Vorkehrungen und ohne spezielle Trägerstoffe in üblichen Sprühtürmen hergestellt werden kann.

Ein weiteres Ziel der Erfindung war die Verwendung leicht zugänglicher preiswerter Rohstoffe in Form von Nebenprodukten der Milchindustrie anstelle von reinen Kohlehydraten.

Die erfindungsgemäße Aufgabe wurde mit einem Verfahren zur Herstellung eines ein Eiweißkolloid und ein Disaccharid enthaltenden Vitamin-E-Trockenpulvers in der Weise gelöst, daß man Vitamin-E-ester in einer an Erdalkali-Ionen-armen und an Lactose-reichen Restflüssigkeit der Milchzuckergewinnung, unter Zusatz von 2 bis 30 Gew.%, bezogen auf die Trockenmasse der Restflüssigkeit, Caseinat dispergiert und die Dispersion sprühtrocknet. Man erhält freifließende Vitamin-E-Trockenpulver, die einen angenehmen Geschmack haben und als Zusatz zu Futter- und Lebensmitteln geeignet sind, außerdem weisen die Trockenpulver gute Tablettierungseigenschaften auf.

Als Vitamin-E-ester kommen übliche Ester des d- und d,l-$\alpha$-Tocopherols in Betracht. Im einzelnen seien genannt: Vitamin-E-Acetat, Vitamin-E-Succinat, Vitamin-E-Palmital, Vitamin-E-Nicotinat. Hiervon ist das Acetat bevorzugt.

Die Vitamin-E-ester wendet man in solchen Mengen an, daß der Gehalt im Trockenpulver 10 bis 60 Gew.% beträgt. Bevorzugte Vitamin-E-Gehalte im erfindungsgemäß hergestellten Trockenpulver sind 20 bis 50 Gew.%.

Unter an Erdalkali-Ionen-armen und an Lactose-reichen Restflüssigkeiten der Milchzuckergewinnung werden im wesentlichen Milchzucker enthaltende Lösungen verstanden, die bei der Gewinnung von Lactose aus Molke anfallen.

Dies kann einerseits eine nach Abtrennen des Molkeneiweißes aus der Molke, konzentrieren und Verminderung der Erdalkali-Ionen z.B. mit Hilfe der Elektrodialyse erhaltene Lösung sein; vorteilhaft verwendet man aber die überwiegend Lactose enthaltende Restlösung aus der Umkristallisierung von Rohlactose zur Herstellung von Reinlactose. Die erfindungsgemäß zu verwendenden Restlösungen weisen in der Regel einen Gehalt von 10 bis 35 Lactose auf und enthalten neben 0,05 bis 1,0 Gew.% an N-haltigen Substanzen und 0,1 bis 3,0 Gew.% an Mineralsalzen nur Spuren anderer Bestandteile, die sämtlich, da aus der Milch stammend, physiologisch unbedenklich sind. Die genannten Restlösungen, deren Herstellung und Verarbeitung sind im einzelnen von G. Nemitz in Landwirtschaftliche Forschung, Kongressband 1976, Sonderheft 33/11, Seite 300 bis 315 beschrieben.

Als erfindungsgemäß zu verwendende Kolloide kommen vor allem Caseinate in Betracht; diese werden durch Aufschluß von Casein mit alkalisch reagierenden Substanzen, z.B. mittels Natrium- der Kaliumverbindungen der Kohlensäure oder Citronensäure erhalten. Ausgangsmaterial für die Herstellung von Caseinatan ist entweder der sorgfältig gewaschene, vorentwässerte Caseinbruch oder getrocknetes Casein. Durch Alkaliaufschluß bei einem kontrollierten pH-Wert zwischen 6 und 7 wird eine ca 20%ige kolloidale Lösung gewonnen, die im Sprühtrockner oder auf Trockenwalzen getrocknet wird. Im Gegensatz zu Casein sind Caseinate wasserlöslich und deshalb für die Herstellung der erfindungsgemäßen Trockenpulver besonders geeignet. Oft ist es auch zweckmäßig, die Caseinate erst im Naßansatz durch Zugabe von Basen aus Casein herzustellen.

Im einzelnen geht man zur Herstellung der Vitamin-E-Trockenpulver in der Regel so vor, daß man das Caseinat, vorzugsweise das Natrium- oder Kaliumcaseinat in der Lactose enthaltenden Restflüssigkeit zweckmäßig unter Einhaltung eines pH-Wertes von 6 bis 8 löst. Mit Hilfe eines Emulgators z.B. Fettsäuremonoglycerids, Fettsäurediglycerids oder Glycerinpolyäthylenglykolricinoleats wird dan das ölige Vitamin-E-acetat unter Druck homogenisiert und die erhaltene Dispersion in einem Sprühturm sprühgetrocknet.

Im allgemeinen wird die Sprühtrocknung bei

einer Einlaßtemperatur des Trocknungsgases von 100 bis 200°C und einer Auslaßtemperatur von 60 bis 100°C mit Hilfe üblicher, bekannter Vorrichtungen durchgeführt, z.B. einem mit einem Druck-, Fliehkraft- oder Zweistoffdüsenzerstäuber versehenen Sprühtrockner. Bei Verwendung eines Sprühtrockners mit Fliehkraftzerstäuber wird die Zerstäubung in der Regel bei 10 000 bis 25 000 UpM durchgeführt.

Die Wahl der Mengen des Caseinat ist von dem Gehalt des Vitamin E im herzustellenden Trockenpulver abhängig. So wird bei Herstellung eines Trockenpulvers mit hohem Vitamin-E-acetat-Gehalt, z.B. 50 Gew.%, der Gehalt an Caseinat 3 bis 30 Gew.%, bezogen auf das Trockenpulver, und bei Herstellung eines Trockenpulvers mit relative niedrigem Gehalt an Vitamin-E-acetat, z.B. 25 Gew.%, der Gehalt an Caseinat 2 bis 20 Gew.%, bezogen auf das Trockenpulver, betragen. Der Rest ist jeweils im wesentlichen Lactose.

Die erfindungsgemäßen Trockenpulver können neben Vitamin E noch weitere Stoffe enthalten, z.B. weitere Vitamine, sowie andere in Futter- und Lebensmittel erwünschte Wirkstoffe, sowie gegebenenfalls technische Hilfsstoffe üblicher Art, z.B. Fleißhilfsmittel wie synthetische Kieselsäure (vgl. Synthetische Kieselsäure als Hilfsstoff in der Futtermittelindustrie "Kraftfutter" 53, S. 436-450 (1970)).

### Beispiel 1

In 1 000 kg Lactoserestlösung der Milchzuckergewinnung mit einem Fesstoffgehalt von 27% werden 59,5 kg Casein bei einer Temperatur von 60-65°C dispergiert. Durch Zugabe von 10%-iger Natronlauge wird ein pH-Wert von 6,5 eingestellt. 418 kg Vitamin-E-Acetat (93,6% an d,l-$\alpha$-Tocopherolacetat durch GC-Bestimmung) werden mit 25 kg eines Fettsäuremonoglycerids mit einem Druckhomogenisator mit der erhaltenen Lösung bei 120 bar homogenisiert. Die so hergestellt Vitamindispersion wird in einem mit Düsen ausgestatteten Sprühtrockner mit einer Trockenlufttemperatur von 170°C und einer Ablufttemperatur von 90°C auf eine Restfeuchte von 1,1% getrocknet. Währen des Sprühvorgangs wird kontinuierlich ca. 1% amorphe Kieselsäure, bezogen auf das Trockengewicht, zur Verbesserung der Fließfähigkeit zudosiert. Man erhält 753 kg eines Produkts, dessen Vitamin-E-Gehalt 50,7% beträgt, bestimmt durch Gaschromatographie.

### Biespiel 2

In 1000 kg Lactoserestlösung mit einem Feststoffgehalt von 27% werden 23 kg Casein bei 60-65°C dispergiert. Der pH-Wert wird durch Zugabe von 10%-iger Natronlauge auf 6,5 eingestellt. 116 kg Vitamin-E-Acetat (93,6% an d,l-$\alpha$-Tocopherolacetat durch GC-Bestimmung) werden mit 7 kg eines Fettsäuremonoglycerids wie in Beispiel 1 homogenisiert und sprühgetrocknet bis zu einer Restfeuchte von 2,1%. Man erhält 408 kg Trockenpulver mit 26% Vitamin-E-Acetat, bestimmt durch Gaschromatographie.

### Beispiel 3

In 1000 kg Lactoserestlösung mit einem Feststoffgehalt von 27% werden 82 kg Casein bei 60-65°C dispergiert. Durch Zugabe von 10%-iger Natronlauge wird der pH-Wert auf 6,5 eingestellt. 138 kg Vitamin-E-Acetat (93,6% and d,l-$\alpha$-Tocopherolacetat durch GC-Bestimmung) werden mit 8,5 kg eines Fettsäuremonoglycerids wie in Beispiel 1 homogenisiert und sprühgetrocknet bis zu einer Restfeuchte von 1,6%. Man erhält 515 kg Trockenpulver mit 25,1% Vitamin-E-Acetat, bestimmt durch Gaschromatographie.

### Patentansprüche

1. Verfahren zur Herstellung eines Eiweißkolloid und ein Disaccharid enthaltenden Vitamin-E-Trockenpulvers, das 10 bis 60 Gew.% Vitamin-E-Ester enthält, unter Verwendung der Sprühtrocknung, dadurch gekennzeichnet, daß man Vitamin-E-Ester in einer Restflüssigkeit der Milchzuckergewinnung, die einen Gehalt von 10 bis 35% Lactose aufweist und neben 0,05 bis 1,0 Gew.% an N-haltigen Substanzen und 0,1 bis 3,0 Gew.% an Mineralsalzen nur Spuren anderer, aus der Milch stammender Bestandteile enthält, unter Zusatz von 2 bis 30 Gew.%, bezogen auf die Trockenmasse der Restflüssigkeit, Caseinat dispergiert und die Dispersion sprühtrocknet.

2. Verfahren gemäß Anspruch 1, dadurch gekennzeichnet, daß man Vitamin-E-Acetat verwendet.

3. Verfahren gemäß Anspruch 1, dadurch gekennzeichnet, daß man Restflüssigkeiten aus der Reinigung von Roh-Lactose verwendet.

4. Verfahren gemäß Anspruch 1, dadurch gekennzeichnet, daß man das Caseinat unter Einhaltung eines pH-Wertes von 6 bis 8 in der Lactose-reichen Restflüssigkeit auflöst.

### Claims

1. A process for the preparation, by spray-drying, of a vitamin E dry powder, containing a protein colloid and a disaccharide and from 10 to 60% by weight of vitamin E ester, wherein a vitamin E ester is dispersed in a residual liquor from the production of lactose, which contains from 10 to 35% of lactose and, apart from 0.05 to 1.0% by weight of nitrogenous substances and from 0.1 to 3.0% by weight of mineral salts, only traces of other constituents originating from milk, in the presence of from 2 to 30% by weight, based on the solids content of the residual liquor, of a caseinate, and the dispersion is spray-dried.

2. A process as claimed in claim 1, wherein vitamin E acetate is used.

3. A process as claimed in claim 1, wherein a residual liquor from the purification of crude lactose is used.

4. A process as claimed in claim 1, wherein the caseinate is dissolved in the lactose-rich residual liquor whilst maintaining the pH at from 6 to 8.

**Revendications**

1. Procédé pour la préparation d'une poudre sèche de vitamine E contenant un colloïde albumineux et un diholoside, poudre qui contient 10 à 60% en poids d'ester de vitamine E, avec utilisation du séchage par pulvérisation, caractérisé en ce qu'on met en dispersion un ester de vitamine E dans un liquide résiduel de l'extraction du sucre de lait qui présente une teneur en lactose de 10 à 35% et ne contient, outre 0,05 à 1,0% en poids de substances azotées et 0,1 à 3,0% en poids de sels minéraux, que des traces d'autres éléments provenant du lait, avec addition de 2 à 30% en poids de caséinate par rapport à la matière sèche du liquide résiduel, en en ce qu'on sèche par pulvérisation la dispersion.

2. Procédé selon la revendication 1, caractérisé en ce qu'on utilise l'acétate de vitamine E.

3. Procédé selon la revendication 1, caractérisé en ce qu'on utilise des liquides résiduels provenant de la purification du lactose brut.

4. Procédé selon la revendication 1, charactérisé en ce qu'on dissous le caséinate dans le liquide résiduel riche en lactose en maintenant le pH à une valeur comprise entre 6 et 8.